(19) Europäisches Patentamt
European Patent Office
Office européen des brevets

(11) Veröffentlichungsnummer: **0 398 132 B1**

(12) # EUROPÄISCHE PATENTSCHRIFT

(45) Veröffentlichungstag der Patentschrift: **20.09.95**

(51) Int. Cl.⁶: **C07F 9/50**, C07F 15/00, C07B 53/00

(21) Anmeldenummer: **90108686.8**

(22) Anmeldetag: **09.05.90**

Die Akte enthält technische Angaben, die nach dem Eingang der Anmeldung eingereicht wurden und die nicht in dieser Patentschrift enthalten sind.

(54) **Phosphorverbindungen.**

(30) Priorität: **18.05.89 CH 1905/89**
**16.03.90 CH 880/90**

(43) Veröffentlichungstag der Anmeldung:
**22.11.90 Patentblatt 90/47**

(45) Bekanntmachung des Hinweises auf die Patenterteilung:
**20.09.95 Patentblatt 95/38**

(84) Benannte Vertragsstaaten:
**AT BE CH DE DK FR GB IT LI NL**

(56) Entgegenhaltungen:
**EP-A- 0 104 375**
**EP-A- 0 256 634**
**EP-A- 0 269 395**

(73) Patentinhaber: **F. HOFFMANN-LA ROCHE AG**
**Postfach 3255**
**CH-4002 Basel (CH)**

(72) Erfinder: **Cereghetti, Marco, Dr.**
**Wettsteinallee 65**
**CH-4058 Basel (CH)**
Erfinder: **Foricher, Joseph**
**34 Rue Daguerre**
**F-68200 Mulhouse (FR)**
Erfinder: **Heiser, Bernd, Dr.**
**Kalchmattweg 13A**
**D-7854 Inzlingen (DE)**
Erfinder: **Schmid, Rudolf, Dr.**
**Im Weissgrien 24**
**CH-4142 Münchenstein (CH)**

(74) Vertreter: **Cottong, Norbert A. et al**
**Grenzacherstrasse 124**
**Postfach 3255**
**CH-4002 Basel (CH)**

EP 0 398 132 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue, chirale, in (R)- oder (S)-Form vorliegende Phosphorverbindungen der allgemeinen Formel

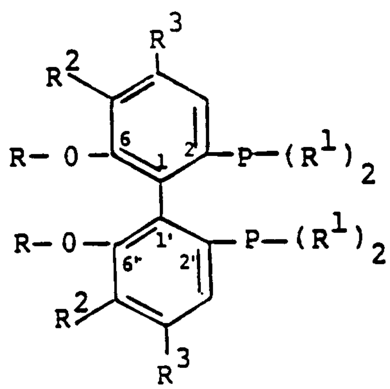

I

worin R niederes Alkyl, $R^1$ Phenyl und $R^2$ und $R^3$ Wasserstoff oder niederes Alkoxy bedeuten.

Die Erfindung betrifft ferner die Herstellung der Phosphorverbindungen der Formel I, sowie deren Verwendung für enantioselektive Reaktionen, wie z.B. asymmetrische Hydrierungen oder auch enantioselektive Wasserstoffverschiebungen in prochiralen, allylischen Systemen.

Der erwähnte Phenylrest kann, im Rahmen der vorliegenden Erfindung, sowohl unsubstituiert als auch in meta- oder para-Stellung oder auch mehrfach substituiert sein. Als Substituenten kommen hier in Frage niedere Alkylgruppen, vorzugsweise Methylgruppen. Der Ausdruck "niederes Alkyl" bedeutet, im Rahmen der vorliegenden Erfindung, geradkettige oder verzweigte Alkylgruppen mit 1 bis 4 Kohlenstoffatomen wie Methyl, Aethyl, Propyl, Isopropyl, n-Butyl, Isobutyl und tert. Butyl. Der Ausdruck "niederes Alkoxy" bedeutet Gruppen, in denen der Alkylrest die vorhergehende Bedeutung hat.

Bevorzugte Phosphorverbindungen der obigen Formel I sind solche, worin R Methyl, $R^1$ unsubstituiertes Phenyl oder in meta- oder para-Stellung Monomethyl-substituiertes Phenyl und $R^2$ und $R^3$ Wasserstoff oder Methoxy bedeuten.

Besonders bevorzugte Verbindungen der Formel I sind:

(R)- oder (S)-(6,6′-Dimethoxybiphenyl-2,2′-diyl)bis(diphenylphosphin)

(R)- oder (S)-(5,5′,6,6′-Tetramethoxybiphenyl-2,2′-diyl)bis(diphenylphosphin)

(R)- oder (S)-(6,6′-Dimethoxybiphenyl-2,2′-diyl)bis(di-p-tolylphosphin)

(R)- oder (S)-(4,4′,5,5′,6,6′-Hexamethoxybiphenyl-2,2′-diyl)bis(diphenylphosphin)

Die erfindungsgemässen Verbindungen der Formel I können dadurch hergestellt werden, dass man eine Verbindung der Formel

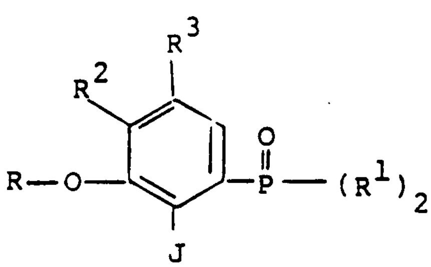

II

worin R, $R^1$, $R^2$ und $R^3$ obige Bedeutung haben,

einer Ullmann-Kopplung unterwirft, dass man eine so erhaltene, in der (RS)-Form vorliegende Verbindung der Formel

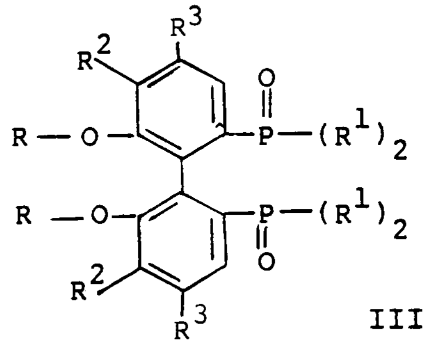

III

worin R, $R^1$, $R^2$ und $R^3$ obige Bedeutung haben,
mittels Dibenzoylweinsäure oder Di-p-Toluylweinsäure in die (R)- und (S)-Form auftrennt, und diese anschliessend reduziert.

Die Ueberführung einer Verbindung der Formel II in eine, in (RS)-Form vorliegende Verbindung der Formel III erfolgt erfindungsgemäss mittels einer Ullmann-Kopplung. Es handelt sich hierbei um eine an sich bekannte Reaktion, welche unter den hierfür üblichen Bedingungen durchgeführt werden kann. So kann diese Reaktion beispielsweise durch Erhitzen einer Verbindung der Formel II in einem inerten organischen Lösungsmittel, wie z.B. N,N-Dimethylformamid, mit z.B. mit Jod aktiviertem Kupferpulver auf eine Temperatur von etwa 110 °C bis etwa 200 °C, durchgeführt werden. Gegebenenfalls kann die Reaktion auch ohne Lösungsmittel, d.h. in der Schmelze durchgeführt werden.

Die als Ausgangsmaterial verwendeten Verbindungen der allgemeinen Formel II sind neu und somit ebenfalls Gegenstand der vorliegenden Erfindung. Sie können beispielsweise dadurch hergestellt werden, dass man eine Verbindung der allgemeinen Formel

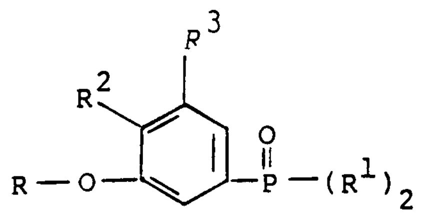

IV

worin R, $R^1$, $R^2$ und $R^3$ obige Bedeutung haben,
einer ortho-Lithiierungs/Jodierungs-Reaktion unterwirft.

Die ortho-Lithiierung einer Verbindung der Formel IV kann in an sich bekannter Weise erfolgen. Beispielsweise kann diese Reaktion durch Umsetzung einer Verbindung der Formel IV mit einem Lithiumamid, z.B. Lithiumdiisopropylamid oder Lithium-2,2,6,6,-tetramethlylpiperidid in Tetrahydrofuran bei einer Temperatur unterhalb -50 °C, vorzugsweise bei etwa -78 °C, erfolgen. Die anschliessende Jodierung kann zweckmässig mit molekularem Jod, mit JCl oder JBr, ebenfalls in Tetrahydrofuran und ebenfalls bei einer Temperatur unterhalb -50 °C, erfolgen.

Die hiervor wiederum als Ausgangsmaterial verwendeten Verbindungen der Formel IV sind bekannte Verbindungen oder Analoge bekannter Verbindungen, welche leicht in an sich bekannter Weise hergestellt werden können [J.J. Monagle et al., J. Org. Chem. 32, 2477 (1967)].

Die Racematspaltung einer in der (RS)-Form vorliegenden Verbindung der Formel III mittels (-)-bzw. (+)-Dibenzoylweinsäure (DBW) oder (-)- bzw. (+)-Di-p-Toluylweinsäure (DTW) kann in für Racematspaltungen von Phosphinoxiden an sich bekannter Weise durchgeführt werden. Dies erfolgt zweckmässig in einem inerten organischen Lösungsmittel und bei einer Temperatur von etwa 0 °C bis etwa 60 °C. Als Lösungsmittel können hier insbesondere genannt werden Chloroform, Methylenchlorid, Essigsäureäthylester, Aceton, Alkohole wie Methanol oder Aethanol und dergleichen, sowie auch Gemische hiervon.

Die so erhaltenenen Addukte der Verbindungen der Formel III mit (-)- bzw. (+)-DBW bzw. DTW können anschliessend, in an sich bekannter Weise, mit einer anorganischen Base behandelt werden, wobei die jeweilige (R)- bzw. (S)-Form der Verbindungen der Formel III freigesetzt werden.

Die Verbindungen der Formel III, und zwar sowohl diejenigen in der (RS)-Form, als auch diejenigen in der (R)- bzw. (S)-Form, sind neue Verbindungen, und als solche ebenfalls Gegenstand der vorliegenden Erfindung.

Die Reduktion einer so erhaltenen, in (R)- oder (S)-Form vorliegenden Verbindung der Formel III kann in an sich bekannter Weise durchgeführt werden. Dies kann beispielsweise mit Silanen, wie z.B. Trichlorsilan, in einem aromatischen Kohlenwasserstoff wie etwa in siedendem Xylol, oder auch in Acetonitril usw., zweckmässig in Gegenwart einer Hilfsbase, wie etwa Triaethylamin, oder vorzugsweise Tributylamin, erfolgen. Gewünschtenfalls kann diese Reduktion auch in einem Autoklaven unter Druck durchgeführt werden.

Gewünschtenfalls kann eine in der (RS)-Form vorliegende Verbindung der Formel III auch vor der Racematspaltung, in zur Reduktion der entsprechenden, in (R)- oder (S)-Form vorliegenden Verbindungen der Formel III, zur entsprechenden (RS)-Verbindung reduziert werden. Diese (RS)-Verbindungen sind ebenfalls neu und Gegenstand der vorliegenden Erfindung.

Sämtliche vorhergehend erwähnten Reaktionen - mit Ausnahme der Racematspaltung - werden zweckmässig unter Inertgas, wie z.B. Argon oder Stickstoff, durchgeführt.

Die erfindungsgemässen Phosphorverbindungen der Formel I bilden Komplexe mit Metallen der Gruppe VIII, insbesondere mit Ruthenium, Rhodium und Iridium, welche als Katalysatoren bei asymmetrischen Hydrierungen und auch für enantioselektive Wasserstoffverschiebungen in prochiralen allylischen Systemen verwendbar sind. Für die erwähnten Hydrierungen sind Ruthenium-Komplexe bevorzugt, während für Isomerisierungen Rhodium-Komplexe bevorzugt sind. Diese Katalysatoren, d.h. die Komplexe aus einem Metall der Gruppe VIII und den Phosphorverbindungen der Formel I sind neu und ebenfalls Gegenstand der vorliegenden Erfindung.

Die in Frage stehenden Komplexe können in an sich bekannter Weise hergestellt werden, z.B. indem man eine Verbindung der Formel I mit einer Verbindung, welche ein Metall der Gruppe VIII abgeben kann, in einem geeigneten inerten organischen oder wässrigen Lösungsmittel umsetzt. Als geeignete, z.B Rhodium abgebende Verbindungen können beispielsweise genannt werden, organische Rhodium-Komplexe mit Aethylen, Propylen und dergleichen, sowie mit bis-Olefinen, z.B. (Z,Z)-1,5-Cyclooctadien, 1,5-Hexadien, Bicyclo[2.2.1]hepta-2,5-dien oder mit weiteren Dienen, welche mit Rhodium leicht lösliche Komplexe bilden. Bevorzugte, Rhodium abgebende Verbindungen sind z.B. Di-$\mu$-chloro-bis[$\eta^4$-(Z,Z)-1,5-cyclooctadien]-dirhodium(I), Di-$\mu$-chloro-bis[$\eta^4$-norbornadien]dirhodium(I), Bis[$\eta^4$-(Z,Z)-1,5-cyclooctadien]rhodium-tetrafluorborat oder Bis[$\eta^4$-(Z,Z)-cyclooctadien]rhodium-perchlorat. Als Iridium abgebende Verbindung kann beispielsweise Di-$\mu$-chloro-bis-[$\eta^4$-(Z,Z)-1,5-cyclooctadien]diiridum(I) genannt werden.

Die in Frage stehenden Ruthenium-Komplexe können durch folgende Formel dargestellt werden

$Ru(Z)_2L$ V

worin Z Halogen oder die Gruppe A-COO, A niederes Alkyl, Aryl, halogeniertes niederes Alkyl oder halogeniertes Aryl und L einen chiralen Diphosphinliganden der Formel I darstellen.

Diese Komplexe können im Prinzip in an sich bekannter Weise hergestellt werden. Zweckmässig und bevorzugt werden Ruthenium-Komplexe beispielsweise dadurch hergestellt, dass man einen Komplex der Formel

$[Ru(Z^1)_2L^1{}_m]_n \cdot (H_2O)_p$ VI

worin $Z^1$ Halogen oder eine Gruppe $A^1$-COO, $A^1$ niederes Alkyl oder halogeniertes niederes Alkyl, $L^1$ einen neutralen Liganden, m die Zahl 1, 2 oder 3, n die Zahl 1 oder 2 und p die Zahl 0 oder 1 darstellen,
mit einem chiralen Diphosphinliganden der Formel I umsetzt, oder, dass man einen Ruthenium-Komplex der Formel

$Ru(CF_3COO)_2L$ VII

worin L einen chiralen Diphosphinliganden der Formel I darstellt,
mit einem das Anion Z abgebenden Salz, worin Z obige Bedeutung hat, umsetzt.

Der Ausdruck "neutraler Ligand" bedeutet im Rahmen der vorliegenden Erfindung, einen leicht austauschbaren Liganden wie etwa ein Diolefin, z.B. Norbornadien, (Z,Z)-1,5-Cyclooctadien usw., oder auch ein Nitril wie Acetonitril, Benzonitril und dergleichen. Falls m die Zahl 2 oder 3 darstellt, können die Liganden gleich oder auch verschieden sein.

Die Rutheniumkomplexe der Formel VI, welche als Ausgangsmaterial verwendet werden, sind bekannte Substanzen oder Analoge bekannter Substanzen, welche leicht in zur Herstellung der bekannten analoger Weise erhalten werden können, beispielsweise gemäss Albers, M.O. et al., J. Organomet. Chem. 272, C62 - C66, (1984).

Die Umsetzung eines Rutheniumkomplexes der Formel VI mit einem chiralen Diphosphinliganden der Formel I kann in an sich bekannter Weise durchgeführt werden. Diese Reaktion kann zweckmässig in einem inerten organischen Lösungsmittel erfolgen. Als Beispiele derartiger Lösungsmittel können genannt werden, z.B. Aether wie Tetrahydrofuran oder Dioxan, Ketone wie etwa Aceton, niedere Alkohole wie etwa Methanol, Aethanol usw., halogenierte Kohlenwasserstoffe wie Methylenchlorid, Chloroform und dergleichen, oder auch Gemische derartiger Lösungsmittel. Die Reaktion kann zudem bei einer Temperatur zwischen etwa O° C und etwa 100°C, und vorzugsweise zwischen etwa 15° C und etwa 60° C erfolgen, jedoch unter striktem Ausschluss von Sauerstoff.

Die Umsetzung eines Rutheniumkomplexes der Formel VII (erhältlich aus einem Komplex der Formel VI) mit einem das Anion Z enthaltenden Salz kann in an sich bekannter Weise erfolgen. Der Ausdruck "ein das Anion Z abgebendes Salz" bedeutet im Rahmen der vorliegenden Erfindung beispielsweise Ammoniumsalze, Alkalimetallsalze oder andere geeignete Metallsalze. Um die Löslichkeit derartiger Salze zu verbessern, können in gewissen Fällen auch Kronenäther oder dergleichen zugesetzt werden.

Die Ruthenium-Komplexe der Formel V können zudem auch noch dadurch hergestellt werden, dass man eine Verbindung der Formel

$(L^2\ Ru\ Cl_2)_2$ VIII

worin $L^2$ einen Aromaten darstellt,
mit einem chiralen Liganden der Formel I umsetzt. Die so erhaltenen Komplexe der Formel

$(L^2\ Ru\ L\ Cl)^+Cl^-$ IX

worin L und $L^2$ obige Bedeutung haben,
können durch Umsetzung mit einem das Anion Z abgebenden Salz, worin Z obige Bedeutung hat, in die Komplexe der Formel V übergeführt werden.

Der Ausdruck "Aromat" bedeutet im Rahmen der vorliegenden Erfindung Benzol, Toluol und dergleichen, oder, vorzugsweise, Cymol.

Die Umsetzung einer Verbindung der Formel VIII mit einem chiralen Liganden der Formel I kann in zur Umsetzung einer Verbindung der Formel VI mit einem chiralen Liganden der Formel I analoger Weise durchgeführt werden. Bevorzugte Lösungsmittel sind in diesem Fall niedere Alkohole, insbesondere Methanol, und die Reaktionstemperatur liegt vorzugsweise bei etwa 40°-50°C. Die Umsetzung einer so erhaltenen Verbindung der Formel IX mit einem das Anion Z abgebenden Salz, kann ebenfalls in zur Umsetzung einer Verbindung der Formel VII mit einen derartigen, das Anion Z abgebenden Salz, analoger Weise erfolgen.

In den Komplexen der Formel IX kann das Anion $Cl^-$ gewünschtenfalls in an sich bekannter Weise auch durch ein anderes, Anion $X^-$, wie etwa $BF_4^-$, $PF_6^-$, $ClO_4^-$ usw., ersetzt werden. Weiterhin kann ein Komplex der Formel IX auch noch, durch Umsetzung mit einem Silbersalz eines wie vorhergehend erwähnten Anions, in Gegenwart eines koordinierenden Solvenses, wie z.B. Acetonitril, in einen Komplex der Formel

$[L^2RU\ L\ S]^{2+}2X^-$ X

worin L und $L^2$ obige Bedeutung haben, $X^-$ ein nicht koordinierendes Anion und S das koordinierende Solvens bedeuten,
übergeführt werden. Diese Ueberführung kann in an sich bekannter Weise erfolgen.

Diejenigen Komplexe der Formel V, worin Z Halogen darstellt, können auch noch dadurch erhalten werden, dass man einen Komplex der Formel

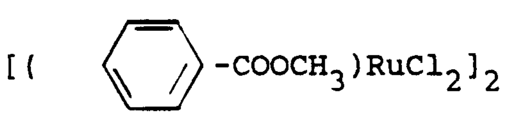 XI

mit einem chiralen Liganden der Formel I umsetzt.

Wie bereits eingangs erwähnt, sind die erfindungsgemässen Phosphorverbindungen, in Form von Komplexen mit Metallen der Gruppe VIII, und insbesondere Ruthenium, u.a. verwendbar für asymmetrische Hydrierungen. Als besonders geeignete Substrate können in diesem Zusammenhang insbesondere genannt werden, Allylalkohole wie z.B. Geraniol, 6,7-Dihydrogeraniol, 6,7-Dihydrofarnesol, 6,7,10,11-Tetrahydrofarnesol und dergleichen, sowie auch $\beta$-Ketoester wie z.B. Acetessigsäuremethyl- oder aethylester usw.

Bei der Durchführung derartiger Hydrierungen, können diese Komplexe zuerst hergestellt und dann zu einer Lösung der zu hydrierenden Substanz gegeben werden. Alternativ können sie jedoch auch in situ hergestellt werden, z.B auch in Gegenwart einer zu hydrierenden Substanz.

Die asymmetrische Hydrierung kann in einem geeigneten, unter den Reaktionsbedingungen inerten organischen Lösungsmittel erfolgen. Als derartige Lösungsmittel können insbesondere genannt werden, niedere Alkohole wie z.B. Methanol oder Aethanol, oder Gemische derartiger Alkohole mit halogenierten Kohlenwasserstoffen wie Methylenchlorid, Chloroform und dergleichen, oder mit cyclischen Aethern wie Tetrahydrofuran oder Dioxan, und dergleichen.

Das Verhältnis von Ruthenium zu Ligand L liegt zweckmässig zwischen etwa 0,5 und etwa 2 Mol, vorzugweise bei etwa 1 Mol Ruthenium pro Mol Ligand. Das Verhältnis von Ruthenium, in den Komplexen der Formel V, zu den zu hydrierenden Substanzen liegt zweckmässig zwischen etwa 0,0005 und etwa 1 Mol %, vorzugsweise zwischen etwa 0,002 und etwa 0,1 Mol %.

Die asymmetrische Hydrierung mit den Komplexen der Formel V erfolgt zweckmässig bei einer Temperatur von etwa 0° C bis etwa 100° C, in Abhängigkeit des verwendeten Substrates. Diese Hydrierung erfolgt zweckmässig auch unter Druck, vorzugsweise bei einem Druck von etwa 5 bis etwa 200 bar, besonders bevorzugt von etwa 30 bis etwa 100 bar.

Weiterhin sind die erwähnten Katalysatoren, wie eingangs erwähnt, verwendbar für enantioselektive Wasserstoffverschiebungen in prochiralen allylischen Systemen. Besonders interessant sind sie z.B. im Zusammenhang mit der Herstellung von optisch aktiven Verbindungen der allgemeinen Formel

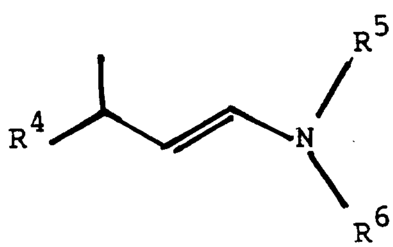

XII

worin $R^4$ geschütztes Hydroxymethyl oder einen Rest der Formel

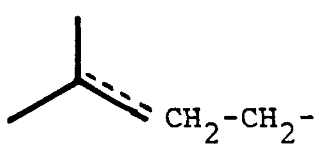

oder

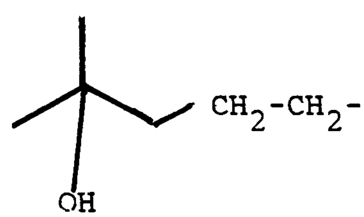

darstellt, worin die gestrichelte Linie eine zusätzliche Bindung darstellen kann, und $R^5$ und $R^6$ niederes Alkyl (1-7 C-Atome) bedeuten,
ausgehend von Verbindungen der allgemeinen Formel

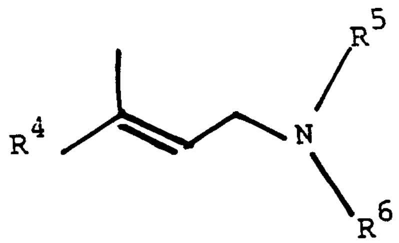

XIII

worin $R^4$, $R^5$ und $R^6$ die obige Bedeutung haben.

Die Verbindungen XII, bzw. die daraus durch Hydrolyse erhaltenen Aldehyde, sowie die von letzteren abgeleiteten Säuren und Alkohole sind z.B. von Interesse als Zwischenprodukte bei der Synthese der Seitenketten der Vitamine E und $K_1$.

Zur Durchführung der erwähnten Wasserstoffverschiebungen, können die Phosphorverbindungen der Formel I als solche, in einer Lösung einer zu behandelnden Verbindung, mit einer z.B. Rhodium oder Iridum abgebenden Verbindung in Kontakt gebracht werden. Andererseits können die Phosphorverbindungen der Formel I zunächst in einem geeigneten Lösungsmittel mit einer z.B. Rhodium oder Iridum abgebenden Verbindung zu dem entsprechenden Katalysator-Komplex umgesetzt werden, und dieser dann zu einer Lösung einer zu behandelnden Verbindung gegeben werden, wobei die letztere Methode bevorzugt ist.

Sowohl die Umsetzung der Phosphorverbindungen der Formel I mit einer z.B. Rhodium oder Iridum abgebenden Verbindung, wie auch die erwähnten Wasserstoffverschiebungen können in geeigneten, unter den Reaktionsbedingungen inerten organischen Lösungsmitteln durchgeführt werden. Als solche können insbesondere genannt werden, niedere Alkanole wie z.B Methanol oder Aethanol, aromatische Kohlenwasserstoffe wie Benzol oder Toluol, cyclische Aether wie Tetrahydrofuran oder Dioxan, Ester wie z.B. Essigester, oder auch Gemische hiervon, und dergleichen. Weiterhin können die Komplexbildungen auch noch in wässrigem Medium oder in Dichlormethan durchgeführt werden.

Das Verhältnis zwischen z.B Rhodium oder Iridium und den Liganden der Formel I liegt zweckmässig zwischen etwa 0,05 und etwa 5 Mol, vorzugsweise zwischen etwa 0,5 und etwa 2 Mol Metall pro Mol Ligand der Formel I.

Die Menge an Metall, in den Komplexen mit den Liganden der Formel I, bezogen auf die zu behandelnden Verbindungen, liegt zweckmässig zwischen etwa 0,005 und etwa 0,5 Mol %, vorzugsweise zwischen etwa 0,01 und etwa 0,2 Mol %.

Die erwähnten Wasserstoffverschiebungen unter Verwendung von Metall-Komplexen mit den Liganden der Formel I können zweckmässig in einem inerten, organischen Lösungsmittel und bei einer Temperatur von etwa Raumtemperatur bis etwa 130 °C durchgeführt werden. Diese Reaktion erfolgt vorzugsweise bei erhöhter Temperatur, d.h. je nach verwendetem Lösungsmittel entweder bei Rückflusstemperatur des Reaktionsgemisches oder in einem geschlossenen Gefäss, unter Druck.

Die folgenden Beispiele dienen zur Illustrierung der Erfindung und stellen in keiner Weise irgendeine Beschränkung dar. In diesen Beispielen haben die gewählten Abkürzungen folgende Bedeutung:

| | |
|---|---|
| GC | : Gaschromatographie |
| MeOBIPHEPO | : (6,6′-Dimethoxybiphenyl-2,2′-diyl)bis- |
| | : (diphenylphosphinoxid). |
| DBW | : O,O′-Dibenzoyl-Weinsäure. |
| DC | : Dünnschichtchromatographie |
| MeOBIPHEP | : (6,6′-Dimethoxybiphenyl-2,2′-diyl)bis- |
| | : (diphenylphosphin) |
| COD | : (Z,Z)-1,5-Cyclooctadien |
| DTW | : 0,0′-Di-p-Toluyl-Weinsäure |
| Tol-MeOBIPHEP | : (6,6′-Dimethoxybiphenyl-2,2′-diyl)bis(di-p-tolylphosphin) |
| $(Me0)_2$BIPHEP | : (5,5′6,6′-Tetramethoxybiphenyl-2,2′-diyl)bis(diphenylphosphin) |
| $(MeO)_3$BIPHEP | : (4,4′,5,5′,6,6′-Hexamethoxybiphenyl-2,2′-diyl)bis(diphenylphosphin). |

Beispiel 1

a) In einem 1,5 L Vierhalssulfierkolben, versehen mit Kühler, Thermometer, Rührer und Aufsatz für Inertgasbegasung, wurden unter Argon 113 g (0,260 Mol) (2-Jod-3-methoxyphenyl)diphenylphosphinoxid und 49,5 g mit Jod aktiviertes Kupferpulver vorgelegt und es wurden 500 ml Dimethylformamid

zufliessen gelassen. Die dunkelbraune Suspension wurde während 1 Stunde auf 140 °C erhitzt (Oelbadtemperatur), wonach gemäss DC-Analyse vollständiger Umsatz eingetreten war. Das abgekühlte Reaktionsgut wurde mit 500 ml Methylenchlorid in einen 2 L Rundkolben transferiert und am Rotationsverdampfer bei 70 °C eingedampft. Der feste, dunkle Rückstand wurde anschliessend mit 500 ml Methylenchlorid versetzt, die Mischung kurz aufgekocht, und nach Abkühlen auf Raumtemperatur wurde vom überschüssigen Kupferpulver und vom gebildeten Kupferjodid abfiltriert. Dann wurde mit 250 ml Methylenchlorid nachgewaschen. Das Filtrat wurde mit 2 x 250 ml gesättigter $NH_4Cl$-Lösung gewaschen, über ca. 100 g Magnesiumsulfat getrocknet, filtriert und eingedampft. Der feste Rückstand (89 g) wurde mit 500 ml Hexan verrührt, die Mischung kurz aufgekocht und heiss abdekantiert. Dieser Vorgang wurde noch 3mal mit je 500 ml, total also mit 1,5 L, Hexan wiederholt. Der feste Rückstand wurde 1 Stunde am Hochvakuum bei 70° C getrocknet, wonach 79,8 g (RS)-(6,6′-Dimethoxybiphenyl-2,2′-diyl)bis-(diphenylphosphinoxid) als leicht bräunliches Kristallisat erhalten wurden. Smp. 307°-308°C. Dieses Material enthielt gemäss NMR noch 0,73 Mol-Aequivalente Methylenchlorid (entsprechend 9,2 Gew %). Die chemische Ausbeute berechnet sich demnach zu 90,7%.

ba) In einem 1,5 L Vierhalssulfierkolben, versehen mit Kühler, mechanischem Rührer und Thermometer, wurden 79,5 g (RS)-MeOBIPHEPO mit 440 ml Methylenchlorid versetzt und die Mischung zum Rückfluss erhitzt, bis eine klare Lösung entstanden war (ca. 2 Stunden). Zu der heissen Lösung wurde unter kräftigem Rühren eine auf 50 °C erhitzte Lösung von 56,3 g (0,157 Mol) (-)-O,O′-Dibenzoyl-L-weinsäure in 520 ml Essigsäureäthylester gegossen, wobei sofort nach Beendigung der Zugabe Kristallisation einsetzte. Die Reaktionsmischung wurde noch 3 Stunden im erkaltenden Oelbad gerührt. Das Kristallgut wurde abgenutscht und mit einem Gemisch von 220 ml Methylenchlorid und 260 ml Essigsäureäthylester gewaschen. Mutterlauge und Waschlösung der Kristallisation wurden beiseite gestellt. Das Kristallgut wurde 4 Stunden bei 80 °C/15 mbar getrocknet, wobei 46,0 g (R)-MeOBIPHEPO/(-)-DBW-Addukt als weisses Kristallisat erhalten wurden; Smp. 209°-210°C; Ausbeute 80,1% d. Th.; $[\alpha]_D^{20}$ = +19,2° (c = 0,8, Aethanol).

bb) Das gemäss ba) erhaltene Material wurde in einem 2 L Erlenmeyer mit Magnetrührstab mit 500 ml Methylenchlorid und 200 ml 2N Natronlauge verrührt, bis aller Festkörper in Lösung gegangen war (30 Min.). Die Phasen wurden getrennt, und die organische Phase wurde mit 200 ml 2N Natronlauge und mit 2 x 250 ml entionisiertem Wasser gewaschen, iber ca. 50 g Magnesiumsulfat getrocknet, filtriert und eingedampft. Der feste Rückstand wurde mit 250 ml Hexan heiss trituriert, nach Abkühlen auf Raumtemperatur abgenutscht und mit 100 ml Hexan gewaschen. Nach Trocknung während 4 Stunden bei 80° C/15 mbar wurden 28,4 g (R)-MeOBIPHEPO als weisses Kristallisat vom Smp. 337,7° C (Thermoanalyse) erhalten; Ausbeute 78,7% d.Th. bezogen auf eingesetztes (RS)-MeOBIPHEPO; $[\alpha]_D^{20}$ = +129,9° (c = 1, $CHCl_3$).

bc) In einem 0,75 L Vierhalssulfierkolben, versehen mit Kühler, Tropftrichter und mechanischem Rührer, wurden die Mutterlauge und Waschlösung aus ba) eingedampft. Der feste Rückstand wurde mit einem Gemisch von 250 ml 2N Natronlauge und 750 ml Methylenchlorid während 30 Min. verrührt. Die organische Phase wurde mit 250 ml 2N Natronlauge und mit 3 x 250 ml entionisiertem Wasser gewaschen, über ca. 50 g Magnesiumsulfat getrocknet, filtriert und am Rotationsverdampfer eingedampft. Der feste Rückstand wurde im Sulfierkolben mit 250 ml Methylenchlorid in der Siedehitze gelöst (1 Stunde). Zu der heissen Lösung wurde unter kräftigem Rühren eine auf 50 °C erhitzte Lösung von 28,8 g (0,08 Mol) (+)-O,O′-Dibenzoyl-D-weinsäure in 275 ml Essigsäureäthylester gegossen, wobei sofort Kristallisation einsetzte. Die Mischung wurde im erkaltenden Oelbad über Nacht gerührt. Das Kristallgut wurde abgenutscht und mit einem Gemisch von 90 ml Methylenchlorid und 110 ml Essigsäureäthylester gewaschen. Nach Trocknung während 1 Stunde bei 100 °C/15 mbar wurden 51 g (S)-MeOBIPHEPO/(+)-DBW-Addukt als weisses Kristallisat vom Smp. 211°-212°C erhalten; Ausbeute 89,6% d.Th. bezogen auf eingesetztes (RS)-MeOBIPHEPO; $[\alpha]_D^{20}$ = -19,4° (c = 1, $C_2H_5OH$). Dieses Material wurde in der gleichen Weise wie im vorangehenden Abschnitt bb) beschrieben weiter behandelt. Dabei wurden 31,8 g (S)-MeOBIPHEPO als weisses Kristallisat vom Smp. 336,5° C (Thermoanalyse) erhalten; Ausbeute 88,4% d.Th. bezogen auf eingesetztes (RS)-MeOBIPHEPO; $[\alpha]_D^{20}$ = -130,4° (c = 1, $CHCl_3$).

ca) In einem 0,75 L Vierhalssulfierkolben, versehen mit Kühler, Thermometer, mechanischem Rührer und 0,25 L Tropftrichter, wurden 27,9 g (R)-MeOBIPHEPO vorgelegt und die Apparatur wurde durch 3maliges Evakuieren/Füllen unter Argon gesetzt. Dann wurden nacheinander unter gutem Rühren 95 ml (0,40 Mol) Tributylamin, 300 ml Xylol-Isomerengemisch und 26 ml Trichlorsilan (34,9 g; 25,7 mMol) einfliessen gelassen. Die milchig-weisse Suspension wurde 3 Stunden am Rückfluss gekocht, wobei das Fortschreiten der Reduktion durch DC kontrolliert wurde. Die erhaltene durchsichtige Lösung wurde auf 0° C gekühlt (Eis/Aethanol-Bad), und es wurden 200 ml 30%-ige Natronlauge unter gutem Rühren so

zugetropft, dass die Temperatur im Reaktionsgefäss 70° C nicht überstieg. Die entstandene milchig-weisse Mischung wurde mit 100 ml Methylenchlorid versetzt, und es wurde solange bei 60° C gerührt, bis zwei klare, separierte Phasen entstanden waren (1 Stunde). Die wässrige Phase wurde mittels einer Spritze entfernt, und die organische Phase wurde nochmals mit 200 ml 30%-iger Natronlauge versetzt und bei 60° C gut durchgerührt. Nach Entfernung der wässrigen Phase wurde die organische Phase mit 100 ml Methylenchlorid in einen Scheidetrichter übergeführt, mit 3 x 200 ml entionisiertem Wasser und mit 200 ml gesättigter NaCl-Lösung gewaschen, über ca. 50 g Magnesiumsulfat getrocknet, filtriert und am Rotationsverdampfer zuerst bei 40° C und dann bei 60° C und schliesslich am Hochvakuum bei 80° C zur Trockne eingedampft. Der feste Rückstand wurde mit 250 ml Aethanol aufgeschlämmt, kurz auf 80°C erhitzt und auf 0° C abgekühlt. Das Kristallgut wurde abgenutscht, mit 250 ml Aethanol und mit 100 ml Pentan gewaschen und 1 Stunde bei 100° C/15 mbar getrocknet. Es wurden 25,75 g (R)-MeOBIPHEP als weisses Pulver vom Smp. 214°C-215°C erhalten; Ausbeute 97,3%; $[\alpha]_D^{20}$ = + 42,4° (c = 1, $CHCl_3$).

Dieses Material wurde unter Argon in 80 ml Toluol heiss gelöst (Oelbadtemp. 110°C) und die heisse Lösung mit 100 ml Aethanol versetzt. Man liess die Mischung über Nacht unter Rühren auf Raumtemperatur abkühlen. Das Kristallisat wurde abgenutscht und zuerst mit einem Gemisch von 80 ml Toluol und 100 ml Aethanol, und dann noch mit 100 ml Pentan gewaschen. Nach Trocknung während 2 Stunden am Hochvakuum bei 110°C wurden 22,8 g (R)-MeOBIPHEP als weisses Kristallisat vom Smp. 214-215° C erhalten; Ausbeute 86,2%;
$[\alpha]_D^{20}$ = +42,3° (c = 1, $CHCl_3$).

cb) 31,5 g (S)-MeOBIPHEPO (51,25 mmol) wurden wie unter Abschnitt ca) beschrieben reduziert. Nach Aufarbeitung wurden 29,3 g (S)-MeOBIPHEP als weisses Pulver vom Smp. 214°-215° C erhalten; Ausbeute 98,1%; $[\alpha]_D^{20}$ = -41,7° (c = 1, $CHCl_3$).

Umkristallisation dieses Materials wie unter ca) beschrieben, lieferte 25,1 g (S)-MeOBIPHEP als weisses Kristallisat vom Smp. 214°-215° C; Ausbeute 84,2%; $[\alpha]_D^{20}$ = -42,5° (c = 1, $CHCl_3$).

d) Das als Ausgangsmaterial verwendete (2-Jod-3-methoxyphenyl)-diphenylphosphinoxid wurde wie folgt hergestellt:

da) In einem 0,35 L Vierhalssulfierkolben, versehen mit Thermometer, Tropftrichter, Rührer und Aufsatz für Inertgasbegasung wurden unter Argon 21,4 g (30 ml; 0,211 Mol) Diisopropylamin und 170 ml trockenes Tetrahydrofuran einfliessen gelassen. Nach Kühlen auf -78°C (Trockeneis/ Aceton-Bad) wurden innerhalb von 15 Minuten 113 ml (0,186 Mol) 1,65N Butyllithium-Lösung in Hexan zugetropft. Das Kühlbad wurde entfernt und die Reaktionsmischung wurde noch 20 Minuten gerührt, wobei die Temperatur bis auf -40°C anstieg. Anschliessend wurde wiederum auf -78°C abgekühlt.

db) In einem wie unter da) beschriebenen 1,5 L Vierhalssulfierkolben wurden 52,5 g (0,170 Mol) (m-Methoxyphenyl)-diphenylphosphinoxid vorgelegt und die Apparatur wurde durch 3maliges Evakuieren/Füllen unter Argon gesetzt. Dann wurden 350 ml trockenes Tetrahydrofuran einfliessen gelassen und die Lösung wurde auf -78°C gekühlt. Unter gutem Rühren wurden die unter da) hergestellten 313 ml (0,186 Mol) Lithiumdiisopropylamid-Lösung innerhalb von 20 Minuten zugetropft, wobei die Temperatur auf ≦ -70°C gehalten wurde. Die Reaktionsmischung verfärbte sich am Anfang der Zugabe rötlich-braun, und gegen Ende der Zugabe bildete sich eine beige Suspension. Nach einer zusätzlichen Rührdauer von 15 Minuten bei -78°C wurde eine Lösung von 47,4 g Jod in 170 ml Tetrahydrofuran innerhalb von 20 Minuten in die Suspension eingetropft, wobei die Reaktionstemperatur auf ≦ -70°C gehalten wurde. (Die Jodlösung war zuvor in einem separaten 250 ml Schlenkrohr unter Argon zubereitet und via Stahlkanüle in den Tropftrichter transferiert worden). Gegen Ende der Zugabe entstand eine rote Lösung, und es setzte die Bildung eines rötlich-braunen, viskosen Materiales ein, welches sich an den Wänden und am Boden des Reaktionsgefässes absetzte. An diesem Punkt wurde der mechanische Rührer abgestellt, das Kühlbad entfernt, und man liess die Reaktionsmischung erwärmen. Bei ca. -30°C entstand wieder eine klare rote Lösung, der Rührvorgang wurde wiederum aufgenommen und man liess auf 0°C erwärmen. Die Reaktionslösung wurde bei 0°C mit einer Lösung von 12 g Natriumthiosulfat-Pentahydrat in 100 ml entionisiertem Wasser versetzt, die Mischung gut durchgerührt, und die Phasen wurden getrennt. Die jetzt gelbliche organische Phase wurde mit 3 x 200 ml gesättigter NaCl-Lösung gewaschen, über ca. 50 g Magnesiumsulfat getrocknet, filtriert und am Rotationsverdampfer bei 40°C bis zur Trockene eingeengt. Der braune Rückstand wurde mit 300 ml tert.-Butyl-methyläther versetzt und die Mischung zum Rückfluss aufgekocht, wobei das Produkt auszukristallisieren begann. Nach Stehen über Nacht bei Raumtemperatur wurde das beige Kristallisat abgenutscht, mit 100 ml tert.-Butyl-methyläther gewaschen und 1 Stunde am Hochvakuum bei 70°C getrocknet. Es wurden 55,7 g (2-Jod-3-methoxyphenyl)-diphenylphosphinoxid als beiges Pulver von Smp. 186°-189°C erhalten.

Beispiel 2

In zu Beispiel 1 analoger Weise wurden folgende Verbindungen hergestellt:

a) (RS)-(5,5',6,6'-Tetramethoxybiphenyl-2,2'-diyl)bis(diphenylphosphinoxid). Smp. 169°-170°C (aus Essigsäureäthylester).

ba) (S)-(5,5',6,6'-Tetramethoxybiphenyl-2,2'diyl)bis(diphenylphosphinoxid)/(-)-DBW-Addukt. (Kristallisation aus Chloroform/Aethanol). $[\alpha]_D^{20}$ = -75° (c = 1, $CH_3OH$).

bb) (S)-(5,5',6,6'-Tetramethoxybiphenyl-2,2'-diyl)bis-diphenylphosphinoxid. Smp. 140°-150°C; $[\alpha]_D^{20}$ = -21,3° (c=1, $CHCl_3$); Smp. 167°-169°C (aus Methylenchlorid/Essigsäureäthylester/Hexan).

bc) (R)-(5,5',6,6'-Tetramethoxybiphenyl-2,2'-diyl)bis(diphenylphosphinoxid. Smp. 140° - 150°C; $[\alpha]_D^{20}$ = +20,6° (c = 1, $CHCl_3$).

ca) (R)-(5,5',6,6'-Tetramethoxybiphenyl-2,2'-diyl)bis(diphenylphosphin). Smp. 171°-172°C; $[\alpha]_D^{20}$ = +5,8° (c = 1, $CHCl_3$).

cb) (S)-(5,5',6,6'-Tetramethoxybiphenyl-2,2'-diyl)bis(diphenylphosphin). Smp. 171°-172°C; $[\alpha]_D^{20}$ = -5,8° (c = 1, $CHCl_3$).

d) Das in diesem Beispiel als Ausgangsmaterial verwendete (2-Jod-3,4-dimethoxyphenyl)-diphenylphosphinoxid wurde wie folgt hersgestellt:

da) 75 g 3,4-Dimethoxyphenyl-diphenylphosphin wurden in 300 ml Methanol aufgenommen. Zu der Suspension wurden 29,3 ml 35% $H_2O_2$-Lösung unter Eisbadkühlung zugetropft. Die klare gelbe Lösung wurde über Nacht bei Raumtemperatur gerührt, dann mit 100 ml ges. $Na_2SO_3$-Lösung und 30 ml 1N HCl versetzt und noch 1 Stunde gerührt. Die entstandene Suspension wurde mit 150 ml Wasser versetzt, und die Lösung wurde am Rotationsverdampfer eingedampft zur Entferung der Hauptmenge des Methanoles. Das ausgefallene gelbe Kristallgut wurde abgenutscht, in $CH_2Cl_2$ aufgenommen, die Lösung über $MgSO_4$ getrocknet, filtriert und eingedampft. Der Rückstand wurde in 450 ml Essigsäureäthylester heiss gelöst und die Lösung mit 450 ml Hexan versetzt. Nach Stehen über Nacht wurde das Kristallisat abgenutscht, mit wenig Hexan gewaschen und bei 0,1 mbar getrocknet. Es wurden 54 g (3,4-Dimethoxyphenyl)diphenylphosphinoxid als leicht beiges Pulver erhalten. Smp. 154,5° -156°C.

db) In einem 0,3 L Vierhalssulfierkolben, ausgerüstet mit Rührer, Thermometer, Tropftrichter und Argonbegasung, wurden 3,38 g (10 mMol) (3,4-Dimethoxyphenyl)diphenylphosphinoxid und 50 ml getrocknetes Tetrahydrofuran vorgelegt. In einem separaten Schlenkrohr wurden unter Argon und unter Rühren 8,0 ml 1,6 N Butyllithiumlösung in Hexan (12,8 mMol) bei -78°C zu einer Lösung von 1,57 g (15,5 mMol) Diisopropylamin in 10 ml Tetrahydrofuran getropft, und die Lösung wurde noch 10 Minuten bei 0°C gerührt. Die so bereitete Lithium-diisopropylamid-Lösung wurde sodann in den Tropftrichter der Reaktionsapparatur transferiert und dann bei ca.- 75°C zu der Lösung des (3,4 -Dimethoxyphenyl)-diphenylphosphinoxides getropft. Die entstehende rosafarbene Suspension wurde noch 2 Stunden bei -78°C gerührt. Dann wurde sie tropfenweise bei < -70°C mit einer Lösung von 3,3 g (13 mMol) Jod in 20 ml Tetrahydrofuran versetzt. Gegen Ende der Zugabe bildete sich eine viskose, schwierig zu rührende Paste, die sich beim anschliessenden Erwärmenlassen auf Raumtemperatur wieder auflöste. Die Reaktionslösung wurde 3 mal mit je 20 ml gesättigter $Na_2S_2O_3$-Lösung und 1 mal mit gesättigter NaCl-Lösung gewaschen, über $Na_2SO_4$ getrocknet, filtriert und eingedampft. Der Rückstand (5 g) wurde an 200 g Kieselgel mit Essigsäureäthylester chromatographiert und das erhaltene Produkt wurde aus tert.-Butyl-methyläther umkristallisiert. Es wurden 3,8 g (2-Jod-3,4-dimethoxyphenyl)-diphenylphosphinoxid als leicht beiges Kristallisat erhalten; Smp. 178,5°-179,5°C.

Beispiel 3

In zu Beispiel 1 analoger Weise wurden folgende Verbindungen hergestellt:

a) (RS)-(6,6'-Dimethoxybiphenyl-2,2'-diyl)bis(di-p-tolylphosphinoxid). Smp. 299°-300° C

ba) (R)-(6,6'-Dimethoxybiphenyl-2,2'-diyl)bis(di-p-tolylphosphinoxid)/(-)-DTW-Addukt. Kristallisation aus Aceton/ $CH_2Cl_2$.

bb) (R)-(6,6'-Dimethoxybiphenyl-2,2'-diyl)bis(di-p-tolylphosphinoxid) $[\alpha]_D^{20}$ = + 117° (c = 1, $CHCl_3$)

bc) (S)-(6,6'-Dimethoxybiphenyl-2,2'-diyl)bis(di-p-tolylphosphinoxid)/(-)-DTW-Addukt. Dieses Addukt wurde durch direkte Kristallisation des Materials aus der Mutterlauge von ba) aus Aceton/ $CH_2Cl_2$ erhalten.

bd) (S)-(6,6'-Dimethoxybiphenyl-2,2'-diyl)bis(di-p-tolylphosphinoxid) $[\alpha]_D^{20}$ = - 112° (c = 1, $CHCl_3$)

ca) (R)-(6,6'-Dimethoxybiphenyl-2,2'-diyl)bis(di-p-tolylphosphin) Smp. 209-210° C; $[\alpha]_D^{20}$ = + 32,3° (c = 0,8, $CHCl_3$)

cb) (S)-(6,6'-Dimethoxybiphenyl-2,2'-diyl)bis(di-p-tolylphosphin) Smp. 208-209° C; $[\alpha]_D^{20}$ = - 32,3° (c = 0,8, $CHCl_3$)

d) Das in diesem Beispiel als Ausgangsmaterial verwendete (2-Jod-3-methoxyphenyl)-di-p-tolylphosphinoxid wurde wie folgt hergestellt.

da) In einem 1.5 l Sulfierkolben mit mechanischem Rührer wurden 7.56 g (0.31 mol) Magnesiumspäne und 300 ml trockenes THF unter Argon vorgelegt, und es wurden 64.0 g (0.342 mol)3-Bromanisol innerhalb von 1 h zugetropft, wobei die Temperatur bis auf 35° anstieg. Nach einer zusätzlichen Rührdauer von 1.5 h wurde mittels eines $CO_2$/Aceton-Bades abgekühlt und eine Lösung von 107.4 g (O.622 mol) Diäthylchlorphosphat in 120 ml THF innerhalb von 1 h zugetropft, wobei die Temperatur bei ≦ -50° gehalten wurde. Die Reaktionsmischung wurde 1 h bei -70°, dann einige h im schmelzenden Eisbad bei 0° und schliesslich über Nacht bei RT gerührt. Zur Aufarbeitung wurde mit Essigsäureäthylester verdünnt, 4mal mit ges. $NaHCO_3$-Lös. und 1 mal mit ges. NaCl-Lös. gewaschen, über $Na_2SO_4$ getrocknet, filtriert und eingeengt. Das erhaltene Oel (75 g) wurde über eine 10 cm Widmer-Kolonne destilliert, wobei 50.9 g 3-Methoxyphenylphosphonsäurediäthylester als leicht gelbliches Oel erhalten wurden. Smp. 118-120°/0,1 mbar.

db) In einem 0.5 l Sulfierkolben wurden 8.35 g (0.345 mol) Magnesiumspäne und 50 ml trockenes THF unter Argon vorgelegt. Dazu wurde eine Lösung von 59.0 g (0.345 mol) p-Bromtoluol in 150 ml THF innerhalb von 1.5 h getropft, wobei die Reaktionstemperatur durch gelegentliches Kühlen (Eisbad) bei 30°-35° gehalten wurde. Anschliessend wurde eine Lösung von 28.0 g (0.114 mol) 3-Methoxyphenylphosphonsäure-diäthylester in 30 ml THF bei RT zugegeben, und das Reaktionsgemisch wurde während 8 h unter Rückfluss gekocht. Zur Aufarbeitung wurde mit ges. $NH_4Cl$-Lösung versetzt, mit Essigsäureäthylester extrahiert, die vereinigten Extrakte mit ges. $NaHCO_3$-Lös. und ges. NaCl-Lös. gewaschen, über $Na_2SO_4$ getrocknet, filtriert und eingedampft. Das erhaltene gelbe Oel wurde aus 200 ml Aether unter Rühren kristallisiert. Das Kristallisat wurde abgenutscht, mit Aether gewaschen und bei 0,1 mbar bei 50° getrocknet. Man erhielt 22,5 g (3-Methoxyphenyl)di-p-tolylphosphinoxid als weisses Pulver. Smp. 96°-98° C.

dc) In einem 1 l Vierhalssulfierkolben versehen mit Rührer, Tropftrichter, Thermometer und Argonbegasung, wurden 16,8 g (50 mMol) (3-Methoxyphenyl)di-p-tolylphosphinoxid in 100 ml trockenem THF gelöst. In einem separaten Schlenk-Rohr wurden unter Argon und unter Rühren 34.0 ml 1.6 N n-Butyllithiumlösung in Hexan (54,4 mMol) bei -78° zu einer Lösung von 9.98 g (70.6 mMol) 2,2,6,6-Tetramethylpiperidin in 50 ml THF getropft. Die Lösung wurde kurzzeitig auf 0° erwärmt, dann wieder auf -78° gekühlt und dann via Kanüle bei ≦ -70° zu der Lösung des Phosphinoxides in THF getropft. Die entstehende rot-braune Lösung wurde noch 30 min bei - 78° gerührt, und dann tropfenweise mit eincr Lösung von 15.0 g (59.1 mMol) Iod in 50 ml THF versetzt, wobei ein beiger Niederschlag gebildet wurde. Man liess das Reaktionsgemisch auf 0° erwärmen, versetzte mit wässriger Natriumpyrosulfit-Lösung und extrahierte mit Essigsäureäthylester. Die Extrakte wurden mit ges. $NaHCO_3$-und ges. NaCl-Lös. gewaschen, über $Na_2SO_4$ getrocknet, filtriert und eingedampft. Der Rückstand wurde an Kieselgel (200 g, Essigsäureäthylester) chromatographiert, wobei 15.3 g (2-Jod-3-methoxyphenyl)-di-p-tolylphosphinoxid erhalten wurden. Nach Umkristallisation aus t-Butylmethyläther erhielt man ein weisses Pulver mit einem Smp. von 146° - 148° C.

## Beispiel 4

In zu Beispiel 1 analoger Weise wurden folgende Verbindungen hergestellt:

a) (RS)-(4,4′,5,5′,6,6′-Hexamethoxybiphenyl-2,2′-diyl)bis(diphenylphosphinoxid). Smp. > 250° C

ba) (R)-(4,4′,5,5′,6,6′-Hexamethoxybiphenyl-2,2′-diyl)bis(diphenylphosphinoxid)/(-)DBW-Addukt. Kristallisation aus $CH_2Cl_2$/Isopropanol $[\alpha]_D^{20}$ = + 34,1° (c = 1, $CHCl_3$)

bb) (R)-(4,4′,5,5′,6,6′-Hexamethoxybiphenyl-2,2′-diyl)bis(diphenylphosphinoxid) Smp. 284° C; $[\alpha]_D^{20}$ = + 51° (c = 1, $CHCl_3$)

bc) (S)-(4,4′,5,5′,6,6′-Hexamethoxybiphenyl-2,2′-diyl)bis(diphenylphosphinoxid) (+) DBW-Addukt.

bd) (S)-(4,4′,5,5′,6,6′-Hexamethoxybiphenyl-2,2′-diyl)bis(diphenylphosphinoxid) Smp. 284° C; $[\alpha]_D^{20}$ = - 51,5° (c = 1, $CHCl_3$)

ca) (R)-(4,4′,5,5′,6,6′-Hexamethoxybiphenyl-2,2′-diyl)bis(diphenylphosphin) Smp. 238° C; $[\alpha]_D^{20}$ = - 2,4° (c = 1, $CHCl_3$)

cb) (S)-(4,4′,5,5′,6,6′-Hexamethoxybiphenyl-2,2′-diyl)bis(diphenylphosphin) Smp. 243° C; $[\alpha]_D^{20}$ = + 2,7° (c = 1,4, $CHCl_3$)

Beispiel 5

Durch Reduktion von (RS)-(6,6′-Dimethoxybiphenyl-2,2′-diyl)bis(diphenylphosphinoxid), von (RS)-(5,5′,6,6′-Tetramethyoxybiphenyl-2,2′-diyl)bis(diphenylphosphinoxid), von (RS)-(6,6′-Dimethoxybiphenyl-2,2′-diyl)bis(di-p-tolylphosphinoxid) bzw. von (RS)-4,4′,5,5'6,6′-Hexamethoxybiphenyl-2,2′-diyl)bis-(diphenylphosphinoxid) in zu Beispiel 1 ca) analoger Weise wurden erhalten:

(RS)-(6,6′-Dimethoxybiphenyl-2,2′-diyl)bis(diphenylphosphin). Smp. 218°-219° C

(RS)-(5,5′,6,6′-Tetramethoxybiphenyl-2,2′-diyl)bis(diphenylphosphin). Smp. 217°-219° C

(RS)-(6,6′-Dimethoxybiphenyl-2,2′-diyl)bis(di-p-tolylphosphin). Smp. 247°-249° C, bzw.

(RS)-(4,4′,5,5′,6,6′-Hexamethoxybiphenyl-2,2′-diyl)bis(diphenylphosphin). Smp. 234,5° C.

Beispiel 6

In einer Glove-Box ($0_2$-Gehalt <1 ppm) wurde ein 500 ml-Autoklav mit 24.0 g (155.6 mMol) Geraniol [-(E)-3,7-Dimethyl-2,6-octadien-1-ol], 120 ml Methanol und 2,9 mg Ru[(R)-MeOBIPHEP]($CF_3C00)_2$ als Katalysator beladen. Die Hydrierung erfolgte bei 20°C und 60 bar. Der Umsatz nach 6 Stunden betrug 99,9 %. Die Hydrierlösung wurde bei 45°C/17 mbar eingedampft und das Rohprodukt bei 60-65°C/0.03 mbar destilliert. Man erhielt 23.7 g (S)-Citronellol [(S)-3,7-Dimethyl-6-octen-1-ol] als farbloses Oel; e.e. 98,9 %.

Zur e.e.-Bestimmung wurde eine Probe mit (R)-6-Methoxy-2,5,7,8-tetramethylchroman-2-carbonsäure verestert und das Diastereomerengemisch gaschromatographisch auf einer Kapillarsäule analysiert.

Das als Katalysator verwendete Ru[(R)-MeOBIPHEP]($CF_3C00)_2$ wurde wie folgt hergestellt:

Eine Lösung von 8,9 g (27.86 mMol) (C0D)Ru(2-Methylallyl)$_2$ in 90 ml Diethylaether wurde bei Raumtemperatur tropfenweise mit 4.3 ml (56.2 mMol) Trifluoressigsäure versetzt. Nach 1-stündigem Rühren bei Raumtemperatur wurde das Solvens abgezogen und der verbleibende Rückstand bei ca. -5°C 2mal mit 5 ml Diäthylaether gewaschen. Das erhaltene gelbe Pulver wurde bei 0,1 mbar getrocknet. Man erhielt 11 g [(COD)Ru($CF_3C00)_2]_2$.

| | | | |
|---|---|---|---|
| $C_{24}H_{24}F_{12}O_8Ru_2$ (870.54) Ber. | C 33.11 | H 2.78 | F 26.19 |
| Gef. | C 33.23 | H 2.86 | F 25.68 |

0,379 g (0,435 mMol) dieses Materials und 0,507 g (0,87 mMol) (R)-MeOBIPHEP wurden mit 8 ml Diäthylaether/THF (3:1 v/v) versetzt und 16 Stunden bei 40°C gerührt. Danach wurde das Solvens abgezogen und der verbleibende gelborange Rückstand 2 mal mit je 5 ml Pentan gewaschen. Nach Trocknen bei 0,1 mbar erhielt man 0,76 g Ru[(R)-MeOBIPHEP]($CF_3COO)_2$ als gelbes Pulver.

$^{31}$P-NMR(202.46 MHz):58.47(s)

Beispiel 7

In einer Glove-Box ($0_2$-Gehalt <1 ppm) wurde ein 500 ml-Autoklav mit 24.0 g (155,6 mMol) Geraniol, 122 ml Methanol und 20.8 mg (o,026 mMol) Ru($CH_3C00)_2$[(R)-MeOBIPHEP] als Katalysator beladen. Die Hydrierung erfolgte bei 20°C und 60 bar. Der Umsatz nach 5 Stunden betrug 99,9 %. Die Hydrierlösung wurde bei 45°C/ 17 mbar eingedampft und das Rohprodukt bei 60-65°C/0,03 mbar destilliert. Man erhielt 23,5 g (S)-Citronellol als farbloses Oel; e.e. 98.8 %.

Zur e.e. Bestimmung wurde eine Probe mit (R)-6-Methoxy-2,5,7,8-tetramethylchroman-2-carbonsäure verestert und das Diastereomerengemisch gaschromatographisch auf einer OV 1 Kapillarsäule analysiert.

Das als Katalysator verwendete Ru($CH_3COO)_2$[(R)MeOBIPHEP] wurde aus dem in Beispiel 6 verwendeten Ru($CF_3COO)_2$[(R)-MeOBIPHEP] wie folgt hergestellt:

Eine Suspension von 0.480 g (0.53 mMol) Ru($CF_3COO)_2$[(R)-MeOBIPHEP] in 5 ml Methanol wurde mit 0.440 g (5.36 mMol) Natriumacetat versetzt und 2 h bei 40° C gerührt. Nach Abziehen des Solvens wurde der Rückstand bei 0.1 mbar getrocknet und anschliessend auf eine Fritte gegeben. Das Produkt wurde mit 15 ml $CH_2Cl_2$ extrahiert, danach das Solvens abgezogen und der Rückstand mit 5 ml Pentan verrührt. Die überstehende Lösung wurde abpipettiert und das verbleibende Pulver i. Vakuum getrocknet. Man erhielt 0,415 g Ru($CH_3C00)_2$[(R)-MeOBIPHEP]

$^{31}$P-NMR ($CDCl_3$, 202.46 MHz): 63.63 (s).

Alternativ zum Vorhergehenden kann der verwendete Katalysator auch noch wie folgt hergestellt werden:

Eine Suspension von 2,58 (2,9 mMol) [$(COD)_2$ $Ru_2$ $(CF_3C00)_4$] ($H_20$) in 20 ml Methanol wurde mit 2.38 g (29.0 mMol) Natriumacetat 1 h bei 40° C gerührt. Nach Abziehen des Solvens wurde der Rückstand bei

0,1 mbar getrocknet und anschliessend auf eine Fritte gegeben. Das Produkt wurde mit 15 ml $CH_2Cl_2$, extrahiert, danach das Solvens abgezogen und der Rückstand 2mal mit je 5 ml Pentan/Diethylaether (5/1 v/v) gewaschen. Der verbleibende gelbe Feststoff wurde im Vakuum getrocknet. Man erhielt 1.24 g (COD)-Ru($CH_3C00)_2$

| | | |
|---|---|---|
| $C_{12}H_{18}O_4Ru$ (327.34) Ber. | C 44.03 | H 5.54 |
| Gef. | C 43.90 | H 5.73 |

0.296 mg (0.90 mMol)(COD)Ru($CH_3COO)_2$ und 0.522 g (0.90 mMol) (R)-MeOBIPHEP wurden in 12 ml Aethanol/THF (3/1 v/v) vorgelegt und 5 h bei 45° C gerührt. Die erhaltene klare rotbraune Lösung wurde im Vakuum eingedampft und der Rückstand unter Rühren 3 mal mit je 10 ml Pentan gewaschen. Nach Trocknen im Vakuum wurden 0.682 g (94.9%) Ru($CH_3COO)_2$ [(R)-MeOBIPHEP] erhalten.

In einer weiteren Alternative kann der verwendete Katalysator auch noch wie folgt hergestellt werden: Eine Lösung von 2.22 g (6,23 mMol) ($\eta^6$-p-Cymol)Ru($CH_3COO)_2$ (hergestellt gemäss D.A. Tocher et al., J. Chem. Soc., Dalton Trans. 1983, 1571-1581, durch Umsetzung von [(p-Cymol)$RuCl_2$] mit $AgO_2CCH_3$ in Toluol) und 3.62 g (6.21 mMol) [(R)-MeOBIPHEP] in 25 ml $CH_2Cl_2$ wurde 48 h bei 50° C gerührt. Anschliessend wurde über eine Fritte filtriert und die erhaltene klare Lösung bis zur Trockene eingedampft. Der Rückstand wurde mit 25 ml Pentan verrührt, abfiltriert und 2 mal mit je 20 ml Pentan gewaschen. Nach Trocknen im Vakuum wurden 4.68 g (93.9 %) Ru($CH_3COO)_2$[(R)-MeOBIPHEP] erhalten.

Beispiel 8

In einer Glove-Box ($O_2$-Gehalt <1 ppm) wurde ein 500 ml-Autoklav mit 24.0 g (155.6 mMol) Geraniol und 20 ml Methanol beladen. Zu dieser Lösung wurde eine Katalysatorlösung - hergestellt durch Lösen von 5.6 mg (0.0064 mMol) [Ru(COD)($CF_3COO)_2]_2$ und 8.3 mg (S)-$(MeO)_2$BIPHEP (0.0129 mMol) in 100 ml Methanol bei Raumtemperatur und Rühren während 16 Stunden - zugegeben. Die Hydrierung erfolgte bei 20°C und 60 bar. Der Umsatz nach 1 Stunde betrug 99.9 %. Die Hydrierlösung wurde bei 45°/17 mbar eingedampft und das Rohprodukt bei 60-65°/0.03 mbar destilliert. Man erhielt 26.8 g (R)-Citronellol als farbloses Oel, e.e. 98.0 %.

Zur e.e.-Bestimmung wurde eine Probe mit (S)-6-Methoxy-2,5,7,8-tetramethylchroman-2-carbonsäure verestert und das Diastereomerengemisch gaschromatographisch auf einer OV 1 Kapillarsäule analysiert.

Beispiel 9

In einer Glove-Box ($O_2$-Gehalt <1 ppm) wurde ein 500 ml-Autoklav mit 27.0 g (175.0 mMol) Geraniol und 136 ml Methanol und 3.38 mg Ru($CF_3C00)_2$[(R)-Tol-MeOBIPHEP] als Katalysator beladen. Die Hydrierung erfolgte bei 20° C und 60 bar. Der Umsatz nach 5 Stunden betrug 100.0 %. Die Hydrierlösung wurde bei 45°/17 mbar eingedampft und das Rohprodukt bei 60-65°/0.03 mbar destilliert. Man erhielt 26.7 g (S)-Citronellol als farbloses Oel; e.e. 99.3 %.

Zur e.e.-Bestimmung wurde eine Probe mit (R)-6-Methoxy-2,5,7,8-tetramethylchroman-2-carbonsäure verestert und das Diastereomerengemisch gaschromatographisch auf einer OV 1 Kapillarsäule analysiert.

Das als Katalysator verwendete Ru($CF_3COO)_2$[(R)-Tol-MeOBIPHEP] wurde in zu Beispiel 6 analoger Weise hergestellt.

Beispiel 10

In einer Glove-Box ($O_2$-Gehalt <1 ppm) wurde ein 500 ml-Autoklav mit 27.0 g (175.0 mMol) Geraniol und 36 ml Methanol beladen. Zu dieser Lösung wurde eine Katalysatorlösung - hergestellt durch Lösen von 6.35 mg (0.0073 mMol) [Ru(COD($CF_3COO)_2]_2$ und 10.25 mg (S)-$(MeO)_3$BIPHEP (0.0146 mMol) in 100 ml Methanol bei Raumtemperatur und Rühren während 16 Stunden - zugegeben. Die Hydrierung erfolgte bei 20°C und 60 bar. Der Umsatz nach 1 Stunde betrug 100.0 %. Die Hydrierlösung wurde bei 45°/17 mbar eingedampft und das Rohprodukt bei 60-65°/0.03 mbar destilliert. Man erhielt 26.8 g (R)-Citronellol als farbloses Oel; e.e. 98.8 %.

Zur e.e.-Bestimmung wurde eine Probe mit (S)-6-Methoxy-2,5,7,8-tetramethylchroman-2-carbonsäure verestert und das Diastereomerengemisch gaschromatographisch auf einer OV 1 Kapillarsäule analysiert.

Beispiel 11

In einer Glove-Box ($O_2$-Gehalt <1 ppm) wurde ein 500 ml-Autoklav mit 27.0 g (175.0 mMol) Geraniol und 136 ml Methanol und 3.6 mg $Ru(CF_3C00)_2$[(S)-$(MeO)_3$BIPHEP] als Katalysator beladen. Die Hydrierung erfolgte bei 20° und 60 bar. Der Umsatz nach 23 Stunden betrug 89.4 %. Die Hydrierlösung wurde bei 45°/17 mbar eingedampft und das Rohprodukt bei 60-65°/0.03 mbar destilliert. Man erhielt 26.4 g (R)-Citronellol, welches nach 10.6 % nicht umgesetztes Geraniol enthielt, als farbloses Oel; e.e. 98.9 %.

Zur e.e.-Bestimmung wurde eine Probe mit (S)-6-Methoxy-2,5,7,8-tetramethylchroman-2-carbonsäure verestert und das Diastereomerengemisch gaschromatographisch auf einer OV 1 Kapillarsäule analysiert.

Das als Katalysator verwendete $Ru(CF_3COO)_2$[(S)-$(MeO)_3$BIPHEP] wurde in zu Beispiel 6 analoger Weise hergestellt.

Beispiel 12

In einer Glove-Box ($0_2$-Gehalt <1 ppm) wurde ein 500 ml-Autoklav mit 33.0 g (145,7 mMol) (2E,7R)-Tetrahydrofarnesol, 170 ml Methanol und 2,65 mg (0,0029 mMol) Ru[(S)-MeOBIPHEP]$(CF_3COO)_2$ als Katalysator beladen. Die Hydrierung erfolgte bei 20°C und 60 bar. Der Umsatz nach 2 Stunden betrug 99,9 %. Die Hydrierlösung wurde bei 45°C/ 17 mbar eingedampft und das Rohprodukt bei 92-93°C/0,01 mbar destilliert. Man erhielt 32,7 g (3R,7R)-Hexahydrofarnesol als farbloses Oel; 98,7 % e.e. an C(3).

Zur e.e. Bestimmung wurde eine Probe mit (R)-6-Methoxy-2,5,7,8-tetramethylchroman-2-carbonsäure verestert und das Diastereomerengemisch gaschromatographisch auf einer Kapillarsäule analysiert.

Das als Katalysator verwendete $Ru(CF_3COO)_2$[(S)-MeOBIPHEP] wurde in zu Beispiel 6 analoger Weise hergestellt.

Beispiel 13

In einer Glove-Box ($O_2$-Gehalt < 1 ppm) wurde ein 500 ml-Autoklav mit 46,2 g (180 mMol) 3-Oxotetradecansäuremethylester, 131 ml Methanol und einer Lösung von 2,1 mg (0,0036 mMol) (R)-MeOBIPHEP und 1,08 mg (0,0018 mMol) $Ru_2Cl_4(COD)_2(CH_3CN)$ [Lit.: E. Singleton et al., S.- Afr. Tydskr. Chem. 40, 183 (1987)] in 5 ml Dichlormethan beladen. Die Hydrierung erfolgte bei 80°C und 35 bar. Der Umsatz betrug nach 19 Stunden >99%. Die Hydrierlösung wurde bei 50°C/17 mbar eingeengt und der kristalline Rückstand in 500ml Diethylaether aufgenommen. Zur Abtrennung des Katalysators wurde die Aetherlösung über 100 g Kieselgel filtriert; e.e. = 97,1 %. Nach dem Einengen und Trocknen der filtrierten Aetherphase resultierten 45,8 g (R)-3-Hydroxytetradecansäure-methylester als weisse Kristalle; e.e. = 97,1 %. Nach Umkristallisation aus n-Hexan erhielt man 41,9 g reinen (R)-3-Hydroxytetradecansäure-methylester; Smp. 42-43°C; e.e. 99,8 %; $[\alpha]_D^{20}$ :-16,4° (c = 3,8, $CCl_4$).

Zur e.e.-Bestimmung wurde eine Probe mit (S)-6-Methoxy-2,5,7,8-tetramethylchroman-2-carbonsäure verestert und die Diastereomeren gaschromatographisch auf einer Kapillarsäule analysiert.

Beispiel 14

Ein Gemisch von 2,27 g (10,0 mMol) (Z)-N,N-Diaethyl-3,7-dimethyl-2,6-octadienylamin, 36 mg (0,041 mMol) [$\eta^4$-(Z,Z)-1,5-Cyclooctadien][(R)-6,6′-dimethoxybiphenyl-2,2′-diyl)bis(diphenylphosphin)]rhodium(I)-tetra-fluorborat und 5 ml Tetrahydrofuran wurde während 16 Stunden im geschlossenen Rohr auf 85°-90°C erhitzt. Die bräunliche Reaktionsmischung wurde eingedampft und der Rückstand im Kugelrohr bei ca. 150°C/0.2 mbar destilliert, wobei 2.1 g (1E,3R)-N,N-Diaethyl-3,7-dimethyl-1,6-octadienylamin als farbloses Oel erhalten wurden. Das erhaltene Destillat wurde mit Hexan verdünnt, mit 5 ml 50 % Essigsäure versetzt und das Gemisch 1 Stunde gerührt. Die Phasen wurden getrennt, und die organische Phase wurde gewaschen mit Wasser, 1N HCl, gesättigter $NaHCO_3$-Lösung und gesättigter NaCl-Lösung, über $Na_2S0_4$ getrocknet, filtriert und eingedampft. Nach Destillation im Kugelrohr bei ca. 130°C/ 15 mbar wurden 1.38 g (90%) (R)-Citronellol [(R)-3,7-Dimethyl-6-octenal] als farblose Flüssigkeit erhalten.
$[\alpha]_D^{20}$ : + 19,2° (c = 4,6 in $CHCl_3$); e.e = 98 %.

Die enantiomere Reinheit dieses Materials wurde nach Reduktion zum Alkohol in zu Beispiel 13 analoger Weise bestimmt.

Der als Katalysator verwendete Rhodium-Komplex wurde wie folgt hergestellt:

Ein Gemisch von 203 mg (0.50 mMol) Bis [$\eta^4$-(Z,Z)-1,5-cyclooctadien]rhodium(I)-tetrafluoroborat und 291 mg (0.50 mMol) (R)-(6,6′-Dimethoxybiphenyl-2,2′-diyl)bis(diphenylphosphin) in 30 ml Tetrahydrofuran wurde über Nacht bei Raumtemperatur gerührt, wobei ein flockiger, orangefarbener Niederschlag entstand.

Die Mischung wurde am Hochvakuum zur Trockne eingedampft, und der Rückstand wurde mit 30 ml Aether verrührt, abfiltriert, mit wenig Aether gewaschen und am Hochvakuum getrocknet. Es wurden 357 mg [$\eta^4$-(Z,Z)-1,5-Cyclooctadien][(R)-6,6′-dimethoxybiphenyl-2,2′-diyl)bis(diphenylphosphin)]rhodium(I)-tetrafluorborat als oranges Pulver erhalten.

Beispiel 15

In zu Beispiel 14 analoger Weise wurde (Z)-N,N-Diaethyl-3,7-dimethyl-2,6-octadienylamin mit [$\eta^4$-(Z,Z)-1,5-Cyclooctadien][(R)-(6,6′-dimethoxybiphenyl-2,2′-diyl)bis(diphenylphosphin)]rhodium(I)-perchlorat während 48 Stunden bei 65°C umgesetzt. Nach Hydrolyse wurde (R)-Citronellal in 88%iger Ausbeute als farblose Flüssigkeit erhalten:

$[\alpha]_D^{20}$ = + 18,9° (c=5,9, $CHCl_3$); e.e. 98,4 %.

Die enantiomere Reinheit dieses Materials wurde in zu Beispiel 13 analoger Weise bestimmt.

Der als Katalysator verwendete Rhodium-Komplex wurde wie folgt hergestellt:

Durch Umsetzung von 209 mg (0,50 mMol) Bis[$\eta^4$-(Z,Z)-1,5-cyclooctadien]rhodium(I)-perchlorat und 291 mg (0.50 mMol) (R)-(6,6′-Dimethoxybiphenyl-2,2′-diyl)bis(diphenylphosphin) in zu Beispiel 14 analoger Weise, erhielt man 431 mg [$\eta^4$-(Z,Z)-1,5-Cyclooctadien][(R)-(6,6′-dimethoxybiphenyl-2,2′-diyl)bis-(diphenylphosphin)]rhodium(I)-perchlorat als oranges Pulver.

Beispiel 16

Ein Gemisch von 11.37 g (50 mMol) (Z)-N,N-Diaethyl3,7-dimethyl-2,6-octadienylamin, 101,5 mg (0.25 mMol) Bis[$\eta^4$-(Z,Z)-1,5-cyclooctadien]rhodium(I}-tetrafluoroborat, 160 mg (0.275 mMol) (R)-(6,6′-Dimethoxybiphenyl-2,2′-diyl)bis(diphenylphosphin) und 25 ml Tetrahydrofuran wurde unter Rühren während 20 Stunden auf 65°C erhitzt. Die bräunlichrote Reaktionsmischung wurde eingedampft und der Rückstand im Kugelrohr bei ca. 150°C/0.2 mbar destilliert, wobei 10,7 g (1E,3R)-N,N-Diaethyl-3,7-dimethyl-1,6-octadienylamin als leicht gelbliches Oel erhalten wurden. Das Destillat wurde mit 20 ml Hexan verdünnt, mit 50 ml 50% Essigsäure versetzt und das Zweiphasengemisch 1 Stunde kräftig gerührt. Nach Aufarbeitung in zu Beispiel 14 analoger Weise und Destillation im Kugelrohr bei 140°C/15 mbar erhielt man 7,18 g (R)-Citronellal als farblose Flüssigkeit : e.e = 98,6 %;

$[\alpha]_D^{20}$ = + 19,2° (c = 4,75 in $CHCl_3$).

**Patentansprüche**

1. Chirale, in der (R)- oder (S)-Form vorliegende Phosphorverbindungen der allgemeinen Formel

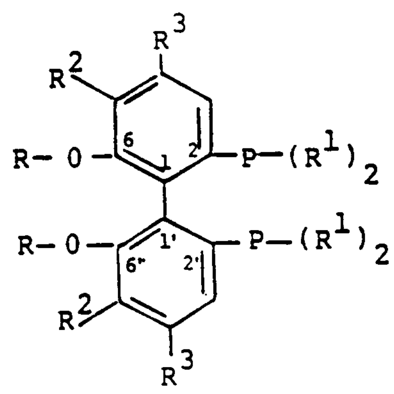

I

worin R niederes Alkyl, $R^1$ Phenyl und $R^2$ und $R^3$ Wasserstoff oder niederes Alkoxy bedeuten.

2. Phosphorverbindungen gemäss Anspruch 1, worin R Methyl, $R^1$ unsubstituiertes oder Monomethyl-substituiertes Phenyl und $R^2$ und $R^3$ Wasserstoff oder Methoxy bedeuten.

3. (R)- oder (S)-(6,6′-Dimethoxybiphenyl-2,2′-diyl)bis(diphenylphosphin).

**4.** (R)- oder (S)-(5,5′,6,6′-Tetramethoxybiphenyl-2,2′-diyl)bis(diphenylphosphin).

**5.** (R)- oder (S)-(6,6′-Dimethoxybiphenyl-2,2′-diyl)bis(di-p-tolylphosphin).

**6.** (R)- oder (S)-(4,4′,5,5′,6,6′-Hexamethoxybiphenyl-2,2-diyl)bis(diphenylphosphin).

**7.** Verfahren zur Herstellung von chiralen, in (R)- oder (S)-Form vorliegenden Phosphorverbindungen der allgemeinen Formel

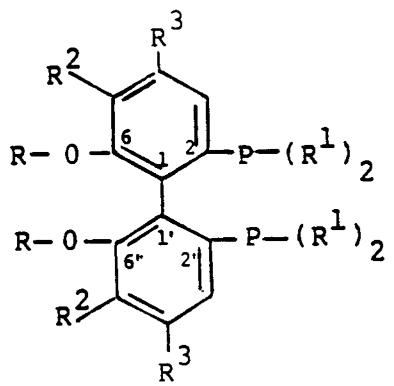

I

worin R niederes Alkyl, $R^1$ Phenyl und $R^2$ und $R^3$ Wasserstoff oder niederes Alkoxy bedeuten, dadurch gekennzeichnet, dass man eine Verbindung der allgemeinen Formel

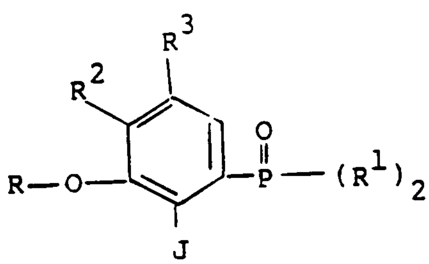

II

worin R, $R^1$, $R^2$ und $R^3$ obige Bedeutung haben,
einer Ullmann-Kopplung unterwirft, dass man eine so erhaltene, in der (RS)-Form vorliegende Verbindung der Formel

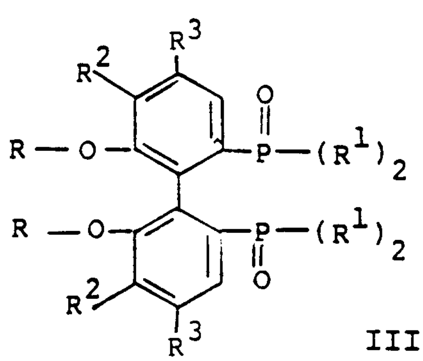

III

worin R, $R^1$, $R^2$ und $R^3$ obige Bedeutung haben,

mittels Dibenzoylweinsäure oder Di-p-Toluylweinsäure in die (R)- und die (S)-Form auftrennt, und diese anschliessend reduziert.

**8.** Komplexe von Phosphorverbindungen der Formel I gemäss Anspruch 1 mit einem Metall der Gruppe VIII.

**9.** Komplexe gemäss Anspruch 8, worin als Metall der Gruppe VIII Ruthenium, Rhodium oder Iridium enthalten ist.

**10.** Komplexe gemäss Anspruch 9, worin als Metall da Gruppe VIII Ruthenium enthalten ist.

**11.** Verwendung der Phosphorverbindungen der Formel I gemäss Anspruch 1, in Form ihrer Komplexe mit einem Metall der Gruppe VIII, als Katalysatoren bei asymmetrischen Hydrierungen und für enantioselektive Wasserstoffverschiebungen in prochiralen, allylischen Systemen.

## Claims

**1.** Chiral phosphorus compounds of the general formula

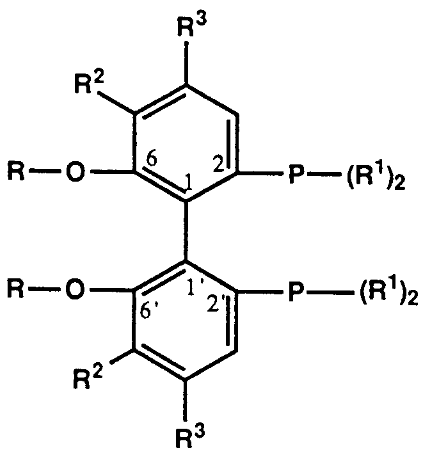

I

wherein R signifies lower alkyl, $R^1$ signifies phenyl and $R^2$ and $R^3$ signify hydrogen or lower alkoxy, which are present in the (R)- or (S)-form.

**2.** Phosphorus compounds according to claim 1, wherein R signifies methyl, $R^1$ signifies unsubstituted phenyl or mono-methyl-substitutedphenyl and $R^2$ and $R^3$ signify hydrogen or methoxy.

**3.** (R)- or (S)-(6,6'-Dimethoxybiphenyl-2,2'-diyl)-bis(diphenylphosphine).

**4.** (R)- or (S)-(5,5',6,6'-Tetramethoxybiphenyl-2,2'-diyl)bis(diphenylphosphine).

**5.** (R)- or (S)-(6,6'-Dimethoxybiphenyl-2,2'-diy)-bis(di-p-tolylphosphine).

**6.** (R)- or (S)-(4,4',5,5',6,6'-Hexamethoxybiphenyl-2,2-diyl)bis(diphenylphosphine).

7. A process for the manufacture of chiral phosphorus compounds of the general formula

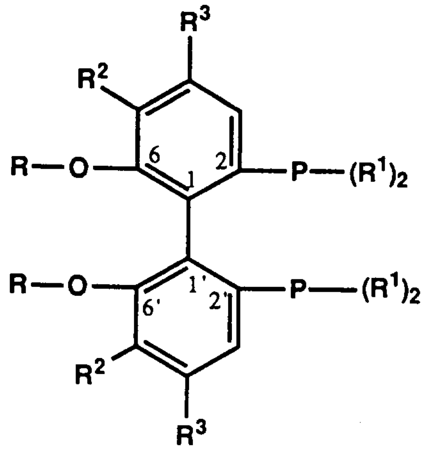

I

wherein R signifies lower alkyl, $R^1$ signifies phenyl and $R^2$ and $R^3$ signify hydrogen or lower alkoxy, which are present in the (R)- or (S)-form, which process comprises subjecting a compound of the general formula

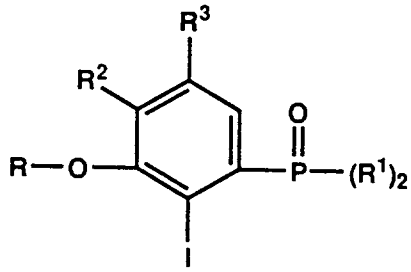

II

wherein R, $R^1$, $R^2$ and $R^3$ have the above significance, to an Ullmann coupling, resolving a thus-obtained compound of the formula

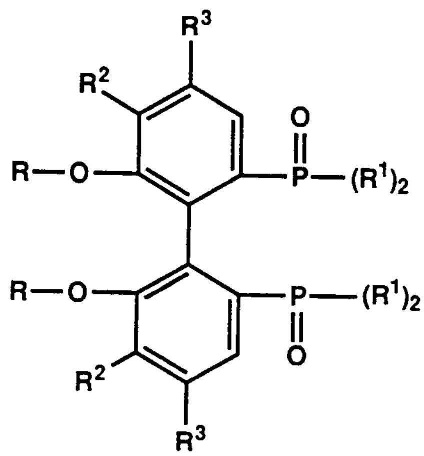

III

wherein R, $R^1$, $R^2$ and $R^3$ have the above significance, which is present in the (RS)-form, by means of dibenzoyltartaric acid or di-p-toluoyltartaric acid into the (R)- and the (S)-form and subsequently reducing this.

8. Complexes of phosphorus compounds of formula I according to claim 1 with a metal of Group VIII.

**9.** Complexes according to claim 8, wherein ruthenium, rhodium or iridium is present as the metal of Group VIII.

**10.** Complexes according to claim 9, wherein ruthenium is present as the metal of group VIII.

**11.** The use of the phosphorus compounds of formula I according to claim 1 in the form of their complexes with a metal of Group VIII as catalysts in asymmetric hydrogenations and for enantioselective hydrogen displacements in prochiral allylic systems.

**Revendications**

**1.** Composés phosphorés chiraux, en configuration (R) ou (S), de formule générale

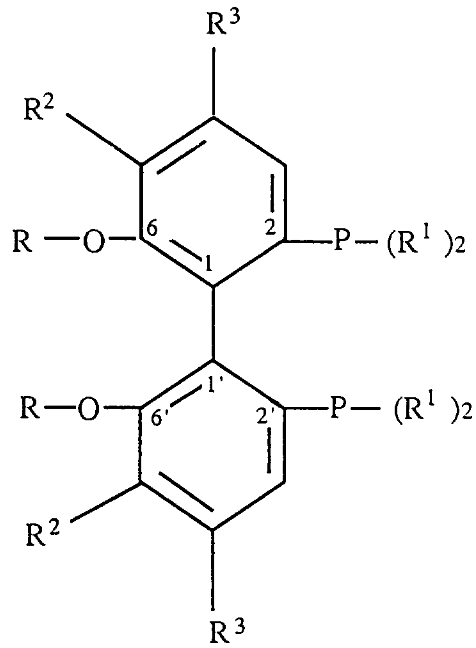

(I)

dans laquelle

- R représente un groupe alkyle inférieur,
- $R^1$ un groupe phényle et
- $R^2$ et $R^3$ l'hydrogène ou des groupes alcoxy inférieurs.

**2.** Composés phosphorés selon la revendication 1, pour lesquels

- R représente un groupe méthyle,
- $R^1$ un groupe phényle non substitué ou monosubstitué par un groupe méthyle et
- $R^2$ et $R^3$ représentent l'hydrogène ou des groupes méthoxy.

**3.** La (R)- ou (S)-(6,6'-diméthoxybiphényl-2,2'-diyl)-bis-(diphénylphosphine).

**4.** La (R)- ou (S)-(5,5',6,6'-tétraméthoxybiphényl-2,2'-diyl)-bis-(diphénylphosphine).

**5.** La (R)- ou (S)-(6,6'-diméthoxybiphényl-2,2'-diyl)-bis-(di-p-tolylphosphine).

**6.** La (R)- ou (S)-(4,4',5,5',6,6-hexaméthoxybiphényl-2,2'-diyl)-bis-(diphényl-phosphine).

**7.** Procédé de préparation de composés phosphorés chiraux, en configuration (R) ou (S), répondant à la formule générale

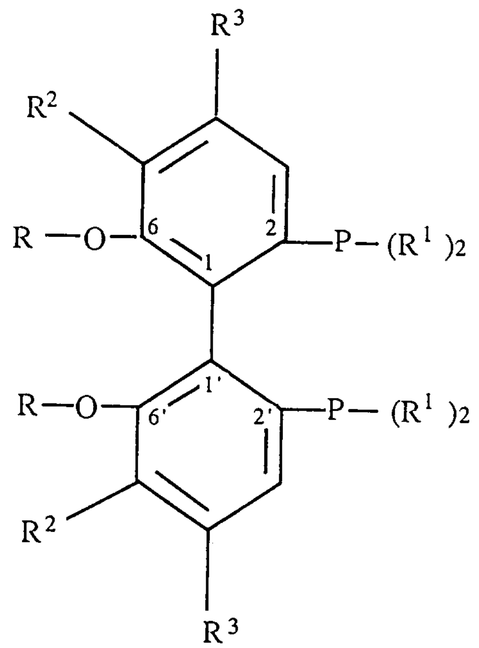

(I)

dans laquelle

- R représente un groupe alkyle inférieur,
- $R^1$ un groupe phényle et
- $R^2$ et $R^3$ l'hydrogène ou des groupes alcoxy inférieurs,

caractérisé en ce que l'on soumet un composé de formule générale

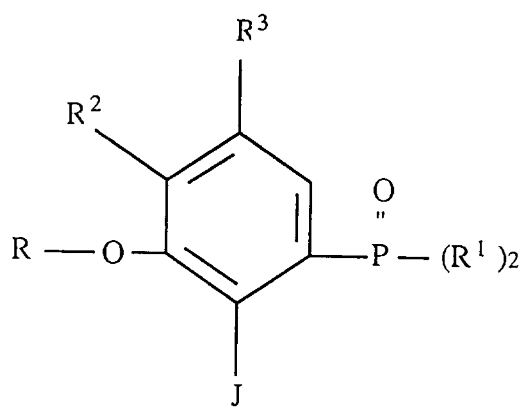

(II)

dans laquelle

R, $R^1$, $R^2$ et $R^3$ ont les significations indiquées ci-dessus, à une condensation d'Ullmann, qui donne un composé de formule

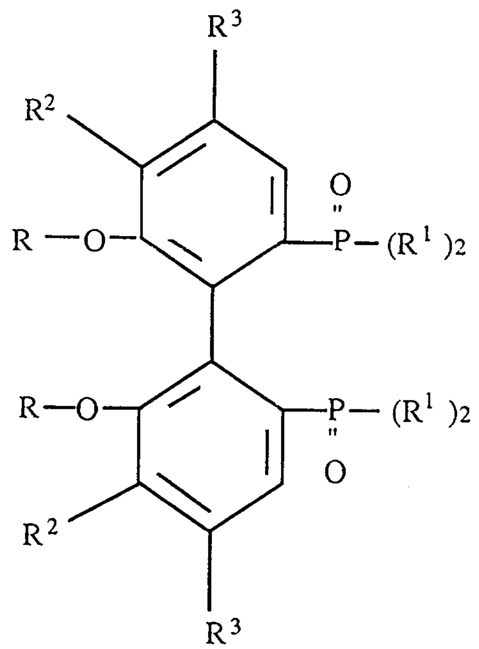

(III)

dans laquelle R, $R^1$, $R^2$ et $R^3$ ont les significations indiquées ci-dessus, en configuration (R, S), qu'on sépare à l'aide de l'acide dibenzoyltartrique ou de l'acide di-p-toluyltartrique en la forme (R) et la forme (S) qu'on soumet ensuite à réduction.

**8.** Complexes des composés phosphorés de formule I de la revendication 1 et d'un métal du groupe VIII.

**9.** Complexes selon la revendication 8, dans lesquels le métal du groupe VIII est le ruthénium, le rhodium ou l'iridium.

**10.** Complexes selon la revendication 9, dans lesquels le métal du groupe VIII est le ruthénium.

**11.** Utilisation des composés phosphorés de formule I de la revendication 1 à l'état de complexes d'un métal du groupe VIII en tant que catalyseurs pour des hydrogénations asymétriques et pour des déplacements énantiosélectifs de l'hydrogène dans des systèmes allyliques prochiraux.